Europäisches Patentamt

European Patent Office

Office européen des brevets

(19)

(11) Publication number: **0 248 632 B1**

(12) # EUROPEAN PATENT SPECIFICATION

(45) Date of publication of patent specification: **15.04.92**   (51) Int. Cl.5: **A61M 5/14**

(21) Application number: **87304858.1**

(22) Date of filing: **02.06.87**

(54) Intravenous fluid flow monitor.

(30) Priority: **06.06.86 US 872199**

(43) Date of publication of application:
**09.12.87 Bulletin 87/50**

(45) Publication of the grant of the patent:
**15.04.92 Bulletin 92/16**

(84) Designated Contracting States:
**CH DE FR GB LI**

(56) References cited:
**EP-A- 0 121 931**
**EP-A- 0 232 599**
**WO-A-87/05224**

(73) Proprietor: **IVAC CORPORATION**
**10300 Campus Pt. Drive**
**San Diego, CA 92121(US)**

(72) Inventor: **Philip, James H.**
**3 Goodnough Road**
**Chestnut Hill MA 02167(US)**

(74) Representative: **Howden, Christopher Andrew et al**
**FORRESTER & BOEHMERT Franz-Joseph-Strasse 38**
**W-8000 München 40(DE)**

# Description

This invention relates to an apparatus for monitoring the flow of intravenous fluid through a parenteral fluid delivery system into a patient's vein. More particularly, the invention relates to apparatus and methods for detecting on a real time basis whether such a fluid is flowing successfully into the patient's vein, whether it is infiltrating the soft tissue surrounding the vein, whether the vein is occluded, whether the vein is phlebitic or whether the cannula has been detached completely from the patient's vein, or if any other disconnection has occurred.

The addition of parenteral fluids to the blood streams of patients by means of cannulas inserted into the veins has been conventional for many years. More recently, parenteral systems have been introduced in which the fluids are positively pumped into the patient's veins, as opposed to earlier systems in which gravity was the motivating force. Pumping has become prevalent because it permits more precise control of fluid flow. Still more recently, systems have been developed in which the pressure in the line connecting the pump to the vein is monitored and a pressure signal is transmitted to a microprocessor or similar device for continuous monitoring of the pressure, so as to determine whether the pressure is within certain limits indicative of proper fluid flow. The goal of such instruments is to provide an indication of whether the cannula is properly in the vein, whether the vein flow is normal, and so on. See, for example, U.S. Patent No. 4,460,355 to Layman, in which a further problem is addressed, that of distinguishing spikes in the fluid pressure due to the discontinuous action of the pump from pressure changes due to abnormal conditions which should be detected so as to give an alarm. The Layman patent provides means for only measuring the pressure of the pumping cycle during certain periods when pressure spikes caused by the pump action may be presumed not to be present.

The applicant is also aware of U.S. patent 4,534,756, assigned to the present assignee, entitled "Fault Detection Apparatus And Method For Parenteral Infusion System." This application broadly discloses a system in which the pressure waveform generated by a pump which produces a pulsating pressure waveform is monitored from cycle to cycle. When a change in the waveform out of preset limits is detected, an alarm is given. While this system was a substantial advance, it frequently could not distinguish fluid flow faults from normal pressure variations particularly at high flow rates.

Accordingly, the art requires an improved intravenous flow monitor which is capable of distinguishing between actual problems, e.g. occlusion of the vein, a cannula becoming detached from the patient's arm, or penetrating through or being withdrawn from the vein so as to be disposed in the surrounding soft tissue, and between artifacts such as relative changes in elevation of the pump and the point of injection of the fluid into the patient.

It is an object of the invention to provide an intravenous flow monitor which can successfully distinguish between real fluid flow problems and artifacts caused by relative vertical motion of the patient's injection point and the pump.

The present inventor has realized that what is needed is an actual measurement of the resistance to flow characteristic of the tissue into which the fluid is being injected. To provide such an indication of resistance to flow provides accurate flow monitoring, regardless of the absolute pressure in the line.

Accordingly, it is an object of the invention to provide an intravenous flow monitor which provides an actual indication of the resistance to flow at the point of insertion of the cannula.

The present inventor has realized further that, given an actual measurement of the resistance to flow, one can then further determine the location of the cannula. For example, the resistance to flow of a phlebitic or occluded vein can be differentiated from the resistance to flow of a normal vein. Similarly, should the cannula penetrate through the vein (a condition referred to as "infiltration"), such that the parenteral fluid is being pumped into the tissue surrounding the vein, this can also be detected. As will be recognized by those skilled in the art, infiltration is a serious condition which can lead to injury. Similarly, a measure of the resistance to flow can readily provide an indication that the catheter has become disconnected.

Accordingly, it is an object of the invention to provide an improved intravenous flow monitor which provides a measure of the resistance to flow of fluid in the line connecting the source of fluid and the cannula, and which furthermore uses such measurement to provide an indication of the conditions at the end of the cannula, i.e. occluded vein, healthy vein, phlebitic vein, disconnection, infiltration, and so forth.

The present invention meets the needs of the art and objects of the invention discussed above by its provision of an improved intravenous pressure monitor in which a peristaltic or other pump is controlled by a microprocessor or an equivalent device to increase and decrease the flow rate, and in which the change in pressure in the fluid line connecting the pump and the patient responsive thereto is monitored.

The present inventor has discovered that when the parenteral fluid flow rate is varied by a relatively small amount, the ratio of the change in pressure to the change in fluid flow rate is the "dynamic resistance" in the line, this term being used to refer to the resistance sensed with respect to a perturbation in flow rate which is small compared to the flow rate and a responsive perturbation in pressure. If resistance is taken to be equal to the thus-computed dynamic resistance, by comparing the resistance in the line to values predetermined by experiment, one can determine the conditions at the cannula. Thus, for example, if the cannula has become detached completely from the patient, there will be substantially no resistance and the pressure will change very little with change in the flow rate. On the other hand, if the cannula has entered an occluded vein, typically the pressure will change significantly with changes in flow rate.

In the preferred embodiment, the microprocessor also monitors the rise time of changes in the pressure; for example, if a square wave change is provided in the flow rate, the corresponding change in the pressure will be somewhat rounded. By monitoring the extent of this rounding, one can differentiate between certain other conditions. For example, the resistance to flow of phlebitic vein in some cases can be substantially equal to the resistance to flow of the soft tissue typically surrounding veins, such that a phlebitic vein could not be distinguished from infiltration merely by monitoring the resistance to flow. However, the inventor has found that the rise time of the pressure changes resulting from square-edged changes in the rate can be used to differentiate between these two conditions.

The invention will be better understood if reference is made to the accompanying drawings in which:

Figure 1 shows a schematic view of the apparatus used to provide intravenous flow monitoring according to the invention;

Figure 2 shows a typical plot of flow rate versus pressure;

Figure 3 show typical signal waveforms, Figure 3A showing changes in the flow rate, and Figure 3B showing corresponding pressure changes;

Figure 4 shows a graph of the absolute value of the resistance in the line compared to the rise time for various conditions; and

Figure 5 shows a flow chart of the more significant steps in the processing performed by the microprocessor.

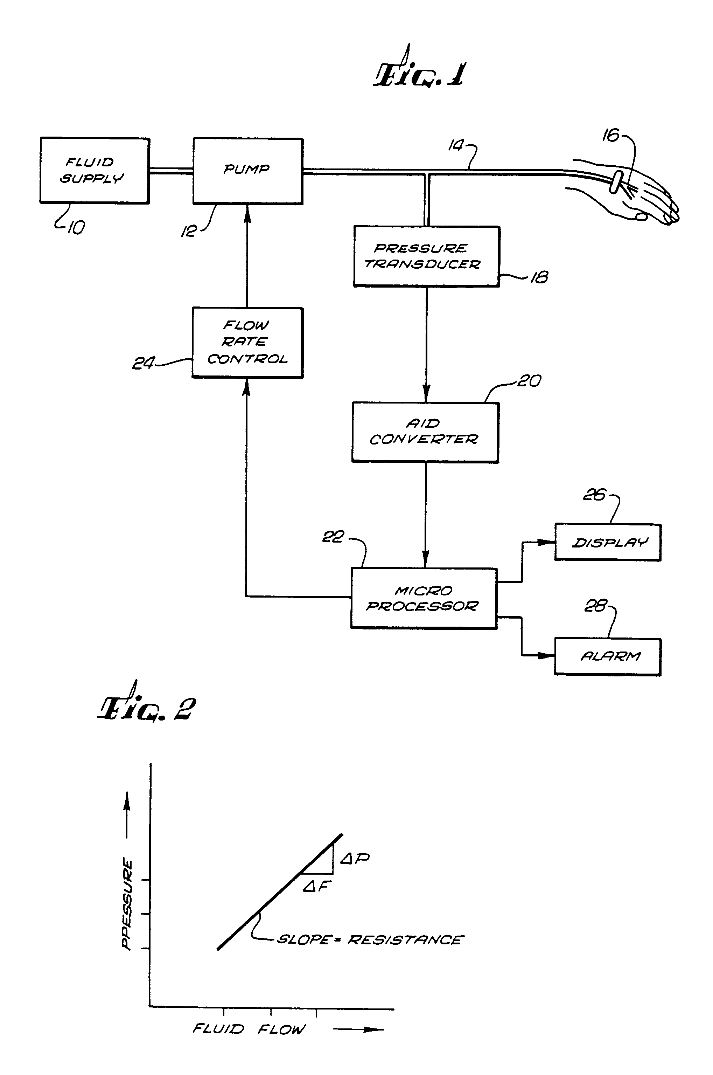

As shown in Figure 1, the system embodying features of the invention generally comprises a fluid supply 10 of any desired parenteral fluid and a pump 12, which may typically be a peristaltic pump to which is connected a tube 14 which in turn is connected to a cannula 16 inserted into the vein of a patient. The pressure in the line 14 is monitored by a pressure transducer 18 which is connected to an analog-to-digital converter 20 for supplying digital data representing the pressure in the tube 14 to a microprocessor 22. The microprocessor 22 provides output signals to a flow rate controller 24 which controls the rate of flow delivered by the pump 12. The microprocessor 22 also provides an operator display 26 and can generate an alarm signal at 28 when certain conditions discussed in detail below are detected. The microprocessor 22 is also enabled to accept operator input for controlling the rate of flow and the like.

According to the present invention, the fluid flow rate is sequentially varied and the difference in pressure caused by the variations in flow rate is noted. By comparing these two values, the instrument can determine the dynamic resistance to flow in the line, which is in turn a measure of the resistance to flow in the environment at the end of the catheter, e.g. in a patient's vein, the tissue surrounding the vein, or the like.

Figure 2 shows a graph of pressure P versus flow rate F and indicates how measurement of the dynamic resistance can be derived. The operator will have set the initial flow rate at some value $F_s$. A corresponding value for the pressure $P_s$ is detected by the pressure transducer 18. The microprocessor then sequentially varies the flow rate F about $F_s$ in one or both directions. The pressure transducer detects corresponding variations in the pressure P as indicated on Figure 2. Two or more points indicating corresponding values for the flow rate and pressure can then be connected, and the slope of the resulting line is equivalent to the dynamic resistance to flow at the end of the cannula.

As described above, this "dynamic resistance," as it is termed herein, is not a measurement of the absolute resistance to flow, because no absolute pressure measurement is made. The measurement in effect is of the variation in resistance to fluid flow variation. The term "resistance" is used in the claims of this application, and should be so understood. The inventor herein has found that a clinically useful measurement is provided.

Figure 3 comprises Figures 3A and 3B, which show typical input fluid flow rate changes and typical detected changes in pressure, respectively. The first condition, represented by the initial change, is for a normal vein; in response to the square edged change in the flow rate, the pressure changes correspondingly. As noted, the change appearing in the pressure waveform is somewhat rounded, having a "rise time" T. This is due to compliance in the lines connecting the pump and the vein, as well as compliance of the normal vein itself.

The second case shown is that of an occlusion in the vein or the connecting tubing. There the change in the flow rate is responded to by a very high change in the pressure, due to the blockage of the occluded vein or tubing.

The third case shown is of an infiltrated vein, that is, one which the cannula has actually penetrated through the vein and is in the soft tissue surrounding the vein. There, the pressure rises substantially, but over a period of time T as the parenteral fluid is pumped into the soft tissue. As is well known, this can be a serious condition leading at least to local or more generalized tissue damage.

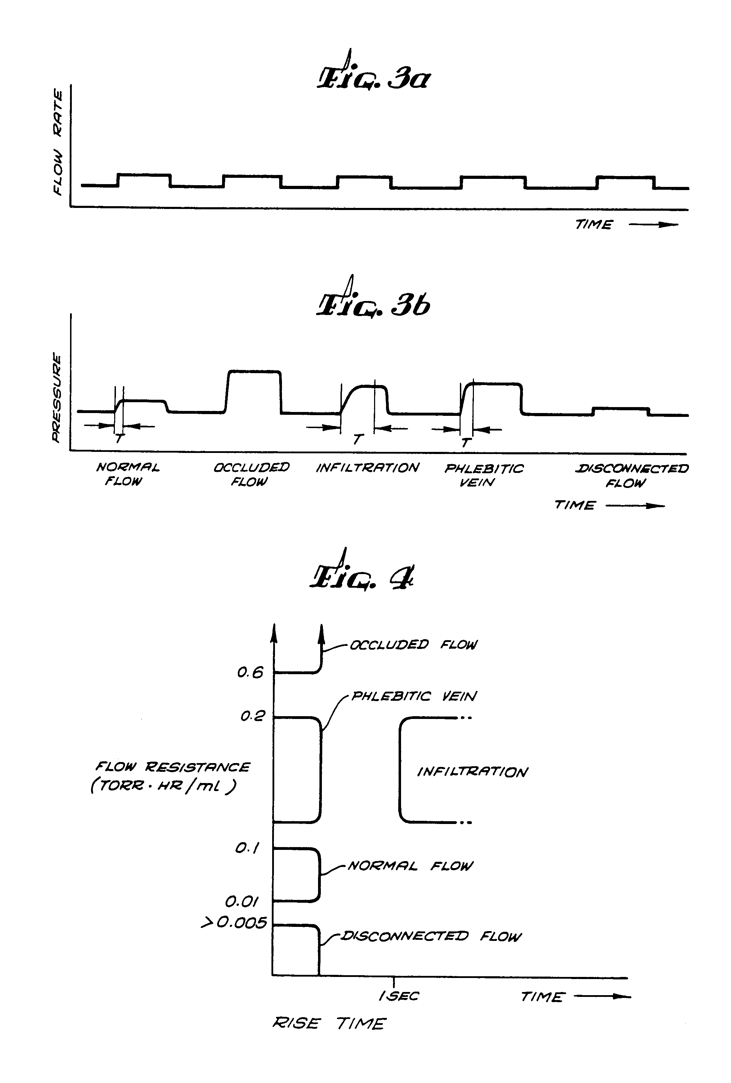

The fourth condition shown is that of a phlebitic vein, in which the vein tends to be stiffer than usual, as well as possibly having some constriction therein. Here, the pressure rise is comparable to that shown in the infiltrated case, but occurs much more quickly, due to the stiffness of the vein. Measurement therefore of the rise time T can provide a way of differentiating between these two conditions, as discussed below in connection with Figure 4.

Finally, the last condition, disconnection, is shown. There, the change in flow rate causes a very minimal change in pressure due to the open-ended cannula or tubing having become disconnected from the patient.

It will be appreciated by those skilled in the art that, if the flow rate change is always the same, as shown in Figure 3A, there is no real need to compare the change in flow rate to the change of pressure, as the pressure change and the rise time T are all that would be needed to distinguish between the various conditions shown. However, as will be discussed below, frequently the flow rate change must vary. Hence it is the better practice to in fact calculate the dynamic resistance R by dividing the amplitude of the pulse in the pressure waveform of Figure 3B into the height of the flow rate pulse of Figure 3A.

Figure 4 shows a plot of actual data, showing the relationship of the absolute value of the dynamic resistance of the vein R, in torr * hr/ml versus the rise time of the pulse T; a number of areas are delineated on the chart to indicate experimental variation and the like. As shown, a disconnected cannula produces very low resistance; a normal vein shows a somewhat higher range of resistance; a phlebitic vein is higher still; and an occluded vein is highest of all. As shown, an infiltration condition gives a value for resistance to flow which is more or less equivalent to that of a phlebitic vein, but has a much higher rise time T.

Accordingly, all that is required to detect the various conditions is to compare the relative resistances to flow and where there is ambiguity, to further examine the rise time of the pressure change.

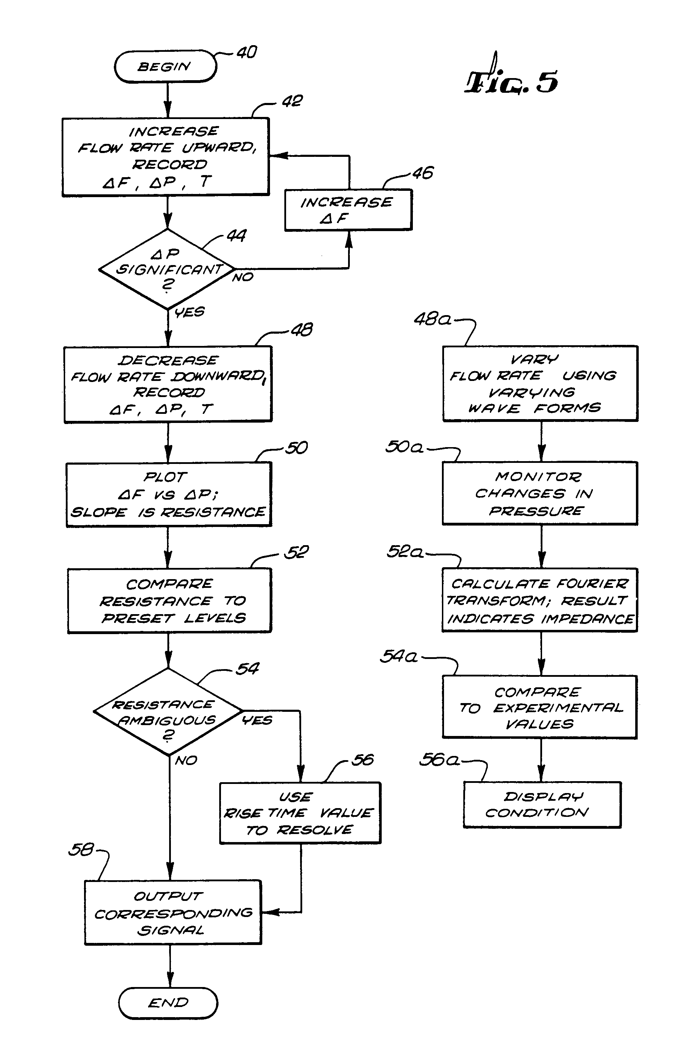

Figure 5 shows the flow chart according to which the microprocessor performs these operations. Processing begins at step 40. The first step 42 is to increase the flow rate and to record a change in flow rate ΔF, a change in pressure ΔP and the rise time T. As step 44, ΔP is compared to certain preset limits. This is because flow rates can vary quite widely, between on the order of millitiers per hour to liters per hour. Accordingly, to simply vary the flow rate by some fixed amount might not always yield a statistically significant change ΔP in the line pressure. Similarly, it may cause non-physiologic extreme changes in pressure. Accordingly, ΔP is compared to preset limits. If ΔP is statistically insignificant, the flow rate change ΔF is altered at step 46 and the process is reperformed. This may be done by the microprocessor automatically, by the operator, or by an interactive combination of both. When suitable values for ΔP are determined, the same procedure is performed by decreasing the flow at step 48 and again recording ΔF, ΔP and T. At step 50, the slope of the line connecting the corresponding values for P and F is calculated. The slope of this line is the resistance R. At step 52, the resistance is compared to preset levels established by experimentation. If the resistance value is ambiguous, as tested at step 54, then the rise time value T is evaluated at step 56. After resolution, if needed, the corresponding output signal is generated at step 58, e.g., on the display 26 (Figure 1).

The rise time T can be simply calculated by programming the microprocessor 22 to take the time derivative of the pressure signal, which would typically be sampled at regular intervals, e.g. every 10 msec. When the derivative is within some predetermined distance from zero, indicating that the pressure pulse has substantially reached its peak, this can be taken as the cut-off point, so that the time between the sending of the flow rate pulse signal to the flow rate controller 24 (Figure 1) and this point is the rise time T. Other well known rise time measurement techniques (e.g., mean transit time measurement techniques) are with the skill of the art.

A further improvement can be made by increasing the sophistication of the signal processing system, e.g., by performing a Fourier analysis in order to determine the information directly relevant to the system's impedance to flow over a spectrum of frequencies. To implement such an approach would typically require use of more complex variation in the flow rate. Sinusiodal and white noise variation may be used, a indicated by step 48a of Figure 5, which substitutes for step 48 according to this alternative embodiment. Steps 50a-56a show the remaining processing steps in this alternative embodiment. In step 50a the pressure in the line is monitored as a function of the change in flow rate. At step 52a, the Fourier transform is taken of this data; the result is the actual impedance to flow of the tissues at the end of the catheter. At step 54a the impedance thus determined is compared to experimentally determined values, and at step 56a the corresponding condition is displayed.

Given the above disclosure, those skilled in the art would have no difficulty implementing the present invention. Note that the main hardware elements of this system, the peristaltic pump 12 controlled by the microprocessor 22 and the pressure transducer 18, are commercially available. However, the microprocessor should be programmed to provide panel displays and/or alarms responsive to the various conditions detected by the system of the invention, and establishing the levels for comparison to the signals. Typical values for R and T are indicated on Figure 4.

While a preferred embodient of the invention has been described, those skilled in the art will recognize that there are additional modifications and improvements which can be made thereto without departure from its spirit and scope. The invention is therefore not to be limited by the above exemplary disclosure, but only by the following claims.

**Claims**

1. A system for monitoring the flow of parenteral fluid through a fluid delivery system (14) to a patient comprising:
   (a) means (10, 12) for supplying parenteral fluid at controllable flow rate through the fluid delivery system to a patient; and
   (b) means (18) for monitoring fluid pressure in the fluid delivery system supplying parenteral fluid to a patient; characterised by
   (c) means (24) for varying the fluid flow rate in a predetermined manner;
   (d) means for determining from the flow rate variations in conjunction with the corresponding variations in fluid pressure in the fluid delivery system, the resistance, as herein defined, to flow in the fluid delivery system, and
   (e) means (22) for comparing the resistance to predetermined limits and for generating output signals responsive thereto.

2. The system of claim 1, wherein the means for calculating the resistance in the fluid delivery system and the means for comparing the resistance to predetermined limits is a microprocessor.

3. The system of claim 2, wherein the microprocessor further comprises means for monitoring the time rate of change of pressure in fluid delivery system which is responsive to changes in flow rate of the fluid.

4. The system of claim 3 wherein the microprocessor analyses the time rate change of pressure in the fluid delivery system and calculates the mean transit time of pulsatile changes in the flow rate of the fluid.

5. The system of the claim 3 wherein the microprocessor analyzes the time rate of change of pressure in the fluid delivery system and calculates a time constant for changes in the pressure which is responsive to changes in the flow rate in the fluid delivery system.

6. The system of claim 1 or claim 2, wherein the means for monitoring the pressure in the fluid delivery system is a pressure transducer outputting an analog signal, and the system further comprises analog-to-digital converter means for conversion of the analog signal to a digital signal for supply to the microprocessor.

7. The system of claim 2, wherein the microprocessor determines the changes in pressure with respect to the changes in flow rate, to provide a resistance value.

8. The system of claim 7, wherein the microprocessor further comprises means for monitoring the time rate of change of pressure.

9. The system of claim 1 wherein the resistance to flow is an impedance.

10. The system of claim 2, wherein the microprocessor further comprises means for monitoring impedance responsive to variations in flow rates, the variations having a predetermined frequency spectrum.

11. The system of claim 10, wherein the microprocessor comprises means for taking the Fourier transform of variations in the pressure which are responsive to the variations in the flow rate.

12. The system of claim 10, wherein said microprocessor further comprises means for calculating the time derivative of changes in the pressure.

13. The system of claim 9, wherein the means for supplying parenteral fluid at controllable flow rates comprises means for causing the flow rates to be varied in a manner having a defined frequency component, and wherein the means for determining the impedance to flow comprises means for analyzing the variations in pressure which are responsive to the variations in flow rates.

14. A method for monitoring the flow of parenteral fluid through a fluid delivery system to a patient comprising:

(a) supplying parenteral fluid at controllable flow rate through the fluid delivery system to a patient; and

(b) monitoring fluid pressure in the fluid delivery system supplying parenteral fluid to a patient characterised by

(c) varying the fluid flow rate in a predetermined manner;

(d) determining from the flow rate variations in conjunction with the corresponding variations in fluid pressure in the fluid delivery system, the resistance, as herein defined, to flow in the fluid delivery system; and

(e) comparing the resistance to predetermined limits and generating output signals responsive thereto.

15. The method of claim 14, wherein the time rate of change of pressure in the fluid delivery system which is responsive to changes in flow rate of the fluid is monitored.

16. The method of claim 15 wherein the time rate of change of pressure in the fluid delivery system is analyzed and the means transit time of pulsatile changes in the flow rate of the fluid is calculated.

17. The method of claim 15, wherein the time rate of change of pressure in the fluid delivery system is analyzed and a time constant for changes in the pressure which is responsive to changes in the flow rate in the fluid delivery system is calculated.

18. The method of claim 15, wherein the pressure in the fluid delivery system is monitored by a pressure transducer outputting an analog signal, and the method further comprises converting the analog signal to a digital signal.

19. The method of claim 15 wherein the changes in pressure with respect to the changes in flow rate are determined in order to determine a resistance value.

20. The method of claim 19, wherein the time rate of change of pressure is monitored.

21. The the method of claim 14, wherein the resistance calculated is an impedance value.

22. The method of claim 21, wherein impedance response to variations in flow rates is monitored, the variations having a predetermined frequency spectrum.

23. The method of claim 22, including determining the Fourier transforms of variations in the pressure which are responsive to variations in the flow rate.

24. The method of claim 23, wherein the time derivative of changes in the pressure is calculated.

25. The method of claim 22, wherein the parenteral fluid is supplied at controllable flow rates which are varied in a manner having a defined frequency content, and the impedance to flow is determined by analyzing the variations in pressure which are responsive to the variations in flow rates.

**Revendications**

1. Système pour surveiller l'administration d'un fluide parentéral par un système d'administration d'un fluide (14) à un patient, comprenant

(a) des moyens (10, 12) pour administrer le fluide parentéral selon un débit contrôlable par la système d'administration de fluide à un patient; et

(b) des moyens (18) pour surveiller la pression du fluide dans le système d'administration de fluide qui administre le fluide parentéral à un patient; caractérisé par

(c) des moyens (24) pour faire varier le débit du fluide d'une manière prédéterminée;

(d) des moyens pour déterminer, à partir des variations du débit et en conjonction avec les variations correspondantes de la pression du fluide dans le système d'administration de fluide, la résistance à l'écoulement, telle qu'elle est définie ici, dans le système d'administration de fluide; et

(e) des moyens (22) pour comparer la résistance avec des limites prédéterminées et pour générer des signaux de sortie sensibles à ces derniers.

2. Système selon la revendication 1, dans lequel les moyens pour calculer la résistance dans le système d'administration de fluide et les moyens pour comparer la résistance à des limites prédéterminées sont constitués par un micro-ordinateur.

3. Système selon la revendication 2, dans lequel le microordinateur comprend dos moyens pour surveiller la vitesse de modification de la pression dans le système d'administration de fluide qui sont sensibles à des modifications du débit du fluide.

4. Système selon la revendication 3, dans lequel le microordinateur analyse la modification dans le temps de la pression dans le système d'administration de fluide et calcule la durée de transit moyenne des modifications pulsatives du débit du fluide.

5. Système selon la revendication 3, dans lequel le microordinateur analyse la vitesse de modification de la pression dans le système d'administration de fluide et calcule une constante de temps pour les modifications de la pression qui est sensible aux modifications du débit dans le système d'administration de fluide.

6. Système selon la revendication 1 ou 2, dans lequel les moyens pour surveiller la pression dans le système d'administration de fluide sont constitués par un transducteur de pression émettant en sortie un signal analogique, et le système comprend en outre des moyens convertisseurs analogiques/numériques pour la conversion du signal analogique en un signal numérique qui est appliqué au micro-ordinateur.

7. Système selon la revendication 2, dans lequel le micro-ordinateur détermine les modifications de la pression par rapport aux modifications du débit, pour déterminer une valeur de résistance.

8. Système selon la revendication 7, dans lequel le micro-ordinateur comprend en outre des moyens pour surveiller la vitesse de modification de la pression.

9. Système selon la revendication 1, dans lequel la résistance à l'écoulement est une impédance.

10. Système selon la revendication 2, dans lequel le micro-ordinateur comprend en outre des moyens pour surveiller l'impédance qui sont sensibles aux variations du débit, les variations ayant un spectre de fréquences prédéterminé.

11. Système selon la revendication 10, dans lequel le micro-ordinateur comprend des moyens pour établir la transformation de fourier des variations de la pression qui sont sensibles aux variations du débit.

12. Système selon la revendication 10, dans lequel ledit micro-ordinateur comprend en outre des moyens calculer la dérivée par rapport au temps des modifications de la pression.

13. Système selon la revendication 9, dans lequel les moyens pour administrer le fluide parentéral selon des débits contrôlables comprennent des moyens pour provoquer une variation des débits d'une manière comportant une composante de fréquence définie, et dans lequel les moyens pour déterminer l'impédance à l'écoulement comprennent des moyens pour analyser les variations de la pression qui sont sensibles aux variations des débits.

14. Procédé pour surveiller l'administration d'un fluide parentéral par un système d'administration de fluide à un patient, comprenant

(a) l'administration de fluide parentéral selon un débit contrôlable par le système d'administration de fluide à un patient; et

(b) la surveillance de la pression du fluide dans le système d'administration de fluide qui administre le fluide parentéral à un patient, caractérisé par:

(c) la modification du débit du fluide selon une manière prédéterminée;

(d) la détermination, à partir des variations du débit en conjonction avec les variations correspondantes de la pression du fluide dans la système d'administration de la résistance à l'écoulement, telle qu'elle est définie dans le système d'administration de fluide; et

(e) la comparaison entre la résistance et des limites prédéterminées et la génération de signaux de sortie sensibles à ces dernières.

15. Procédé selon la revendication 14, dans lequel est surveillée la vitesse de modification de la pression dans le système d'administration de fluide qui est sensible aux modifications du débit du fluide.

16. Procédé selon la revendication 15, dans lequel est analysée la vitesse de modification de la pression dans le système d'administration de fluide et est calculée la durée moyenne de transit des modifications pulsatives du débit du fluide.

17. Procédé selon la revendication 15, dans lequel est analysée la vitesse de modification de la pression dans le système d'administration de fluide et est calculée une constante de temps pour les modifications de la pression qui est sensible aux modifications du débit dans le système d'administration de fluide.

18. Procédé selon la revendication 15, dans lequel la pression dans le système d'administration de fluide est surveillée par un transducteur de pression émettant un signal analogique, et le procédé comprend en outre la conversion du signal analogique en un signal numérique.

19. Procédé selon la revendication 15, dans lequel les modifications de la pression par rapport aux modifications du débit sont déterminées de manière à déterminer une valeur de résistance.

20. Procédé selon la revendication 19, dans lequel la vitesse de modification de la pression est surveillée.

21. Procédé selon la revendication 14, dans lequel la résistance calculée est une valeur d'impédance.

22. Procédé selon la revendication 21, dans lequel la réponse de l'impédance à des variations des débits est surveillée, les variations ayant un spectre de fréquences prédéterminé.

23. Procédé selon la revendication 22, comprenant la détermination des transformées de Fourier des variations de la pression qui sont sensibles aux variations du débit.

24. Procédé selon la revendication 22, dans lequel est calculée la dérivée par rapport au temps des modifications de la pression.

25. Procédé selon la revendication 22, dans lequel le fluide parentéral est administré selon des débits contrôlables qui sont modifiés d'une manière présentant une teneur en fréquence définie, et l'impédance à l'écoulement est déterminée par l'analyse des variations de la pression qui sont sensibles aux variations des débits.

**Patentansprüche**

1. System zum Aufzeichnen des Stromes parenteralen Fluides durch ein Fluidzufuhrsystem (14) zu einem Patienten mit:
   (a) einer Einrichtung (10, 12) zum Liefern parenteralen Fluides mit steuerbarer Durchflußgeschwindigkeit durch das Fluidzufuhrsystem zu einem Patienten; und
   (b) einer Einrichtung (18) zum Überwachen des Fluiddruckes in dem Fluidzufuhrsystem, das parenterales Fluid zu einem Patienten führt;
**gekennzeichnet** durch
(c) eine Einrichtung zum Ändern der Fluid-Durchflußgeschwindigkeit in einer vorbestimmten Weise;
(d) eine Einrichtung zum Bestimmen aus den Änderungen der Durchflußgeschwindigkeit im Zusammenhang mit den entsprechenden Änderungen im Fluiddruck in dem Fluidzufuhrsystem des Stromwiderstandes, wie er hierin definiert ist, in dem Fluidzufuhrsystem und
(e) eine Einrichtung (22) zum Vergleichen des Widerstandes mit vorbestimmten Grenzen und zum Erzeugen von Ausgangssignalen in Reaktion darauf.

2. System nach Anspruch 1, bei dem die Einrichtung zum Berechnen des Widerstandes in dem Fluidzufuhrsystem und die Einrichtung zum Vergleichen des Widerstandes mit vorbestimmten Grenzen ein Mikroprozessor ist.

3. System nach Anspruch 2, bei dem der Mikroprozessor weiterhin eine Einrichtung zum Überwachen des Taktes der Änderungen des Druckes in dem Fluidzufuhrsystem, der auf Änderungen in der Durchflußgeschwindigkeit des Fluides anspricht, aufweist.

4. System nach Anspruch 3, bei dem der Mikroprozessor die Taktänderung des Druckes in dem Fluidzufuhrsystem analysiert und die mittlere Übergangszeit von pulsierenden Änderungen in der Durchflußgeschwindigkeit des Fluides berechnet.

5. System nach Anspruch 3, bei dem der Mikroprozessor die Taktänderung des Druckes in dem Fluidzufuhrsystem analysiert und eine Zeitkonstante für Änderungen in dem Druck berechnet, die auf Änderungen in der Durchflußgeschwindigkeit in dem Fluidzufuhrsystem anspricht.

6. System nach Anspruch 1 oder Anspruch 2, bei dem die Einrichtung zum Überwachen des Druckes in dem Fluidzufuhrsystem ein Druckmeßwandler ist, der ein Analogsignal ausgibt, und das System weiterhin eine Analog/Digital-Wandlereinrichtung zum Umwandeln des Analogsignales in ein Digitalsignal für die Eingabe in den Mikroprozessor aufweist.

7. System nach Anspruch 2, bei dem der Mikroprozessor die Änderungen im Druck in bezug auf die Änderungen der Durchflußgeschwindigkeit bestimmt, um einen Widerstandswert zur Verfügung zu stellen.

8. System nach Anspruch 7, bei dem der Mikroprozessor weiterhin eine Einrichtung zum Überwachen des Taktes von Druckänderungen aufweist.

9. System nach Anspruch 1, bei dem der Stromwiderstand eine Impedanz ist.

10. System nach Anspruch 2, bei dem der Mikroprozessor weiterhin eine Einrichtung zum Überwachen der Impedanz, ansprechend auf Änderungen in der Durchflußgeschwindigkeit, aufweist, wobei die Änderungen ein vorbestimmtes Frequenzspektrum haben.

11. System nach Anspruch 10, bei dem der Mikroprozessor eine Einrichtung zum Aufnehmen der Fourier-Transformation von Änderungen in dem Druck aufweist, die auf die Änderungen in der Durchflußgeschwindigkeit ansprechend sind.

12. System nach Anspruch 10, bei dem der Mikroprozessor weiterhin eine Einrichtung zur Berechnung der Zeitableitung von Änderungen in dem Druck aufweist.

13. System noch Anspruch 9, bei dem die Einrichtung zum Liefern parenteralen Fluides bei steuerbaren Durchflußgeschwindigkeiten eine Einrichtung zum Bewirken, daß die Durchflußgeschwindigkeit in einer Weise geändert wird, daß sie eine bestimmte Frequenzkomponente hat, aufweist, und bei dem die Einrichtung zum Bestimmen der Stromimpedanz eine Einrichtung zum Analysieren der Druckänderungen aufweist, die auf die Änderungen in der Durchflußgeschwindigkeit ansprechen.

14. Verfahren zum Überwachen des Stromes parenteralen Fluides durch ein Fluidzufuhrsystem zu einem Patienten, mit:
(a) dem Liefern parenteralen Fluides bei steuerbarer Durchflußgeschwindigkeit durch das Fluidzufuhrsystem zu einem Patienten; und
(b) dem Überwachen des Fluiddruckes in dem Fluidzufuhrsystem, das das parenterale Fluid zu einem Patienten liefert, **gekennzeichnet** durch
(c) das Variieren der Fluiddurchflußgeschwindigkeit in einer vorbestimmten Weise;
(d) das Bestimmen aus den Änderungen der Durchflußgeschwindigkeit im Zusammenhang mit den entsprechenden Änderungen im Fluiddruck im Fluidzufuhrsystem, dem Stromwiderstand, wie hierin definiert, in dem Fluidzufuhrsystem; und
(e) das Vergleichen des Widerstandes mit vorbestimmten Grenzwerten und das Erzeugen von Ausgabesignalen, die darauf ansprechen.

15. Verfahren nach Anspruch 14, bei dem der Takt von Änderungen des Druckes in dem Fluidzufuhrsystem, der auf Änderungen in der Durchflußgeschwindigkeit des Fluides anspricht, überwacht wird.

16. Verfahren nach Anspruch 15, bei dem der Takt der Änderungen des Druckes in dem Fluidzufuhrsystem analysiert wird und die mittlere Übergangszeit von pulsierenden Änderungen in der Durchflußgeschwindigkeit des Fluides berechnet wird.

17. Verfahren nach Anspruch 15, bei dem der Takt von Änderungen des Druckes in dem Fluidzufuhrsystem analysiert wird und eine Zeitkonstante für Änderungen im Druck, die auf Änderungen in der Durchflußgeschwindigkeit in dem Fluidzufuhrsystem anspricht, berechnet wird.

18. Verfahren nach Anspruch 15, bei dem der Druck in dem Fluidzufuhrsystem durch einen Druckmeßwandler überwacht wird, der ein Analogsignal ausgibt, und das Verfahren weiterhin das Umwandeln des Analogsignales in ein Digitalsignal umfaßt.

19. Verfahren nach Anspruch 15, bei dem die Änderungen im Druck in bezug auf die Änderungen der Durchflußgeschwindigkeit bestimmt werden, um einen Widerstandswert zu bestimmen.

20. Verfahren nach Anspruch 19, bei dem der Takt der Druckänderungen überwacht wird.

21. Verfahren nach Anspruch 14, bei dem der berechnete Widerstand ein Impedanzwert ist.

22. Verfahren nach Anspruch 21, bei dem die Impedanz, ansprechend auf Änderungen in der Durchströmgeschwindigkeit, überwacht wird, wobei die Änderungen ein vorbestimmtes Frequenzspektrum haben.

23. Verfahren nach Anspruch 22, einschließlich des Bestimmens der Fourier-Transformierten von Änderungen in dem Druck, die auf Änderungen in der Durchflußgeschwindigkeit ansprechen.

24. Verfahren nach Anspruch 23, bei dem die Zeitableitung von Änderungen in dem Druck berechnet wird.

25. Verfahren nach Anspruch 22, bei dem das parenterale Fluid bei steuerbaren Durchflußgeschwindigkeiten geliefert wird, die in einer Weise geändert werden, daß sie einen definierten Frequenzgehalt haben und die Stromimpedanz durch Analysieren der Änderungen in dem Druck, die ansprechend auf die Änderungen in der Durchflußgeschwindigkeit sind, bestimmt wird.

## *Fig. 1*

```
┌──────────┐     ┌──────────┐
│  FLUID   │═════│   PUMP   │═══════════════ 14 ─────────────╮  16
│  SUPPLY  │     │          │                                │
└──────────┘     └──────────┘                                │
      └─10           └─12                                     │
                         ↑
              ┌──────────┐        ┌──────────────┐
              │   FLOW   │        │   PRESSURE   │
              │   RATE   │        │  TRANSDUCER  │──18
         24───│ CONTROL  │        └──────────────┘
              └──────────┘               │
                   ↑                     ▼
                   │              ┌──────────────┐
                   │              │     A/D      │──20
                   │              │  CONVERTER   │
                   │              └──────────────┘
                   │                     │
                   │                     ▼              ┌──────────┐
              22───│              ┌──────────────┐   ┌─▶│ DISPLAY  │──26
                   └──────────────│    MICRO     │───┤  └──────────┘
                                  │  PROCESSOR   │   │  ┌──────────┐
                                  └──────────────┘   └─▶│  ALARM   │──28
                                                        └──────────┘
```

## *Fig. 2*

PRESSURE ↑

$\Delta P$
$\Delta F$

SLOPE = RESISTANCE

FLUID FLOW →

*Fig. 3a*

FLOW RATE

TIME →

*Fig. 3b*

PRESSURE

T      T      T

NORMAL      OCCLUDED      INFILTRATION      PHLEBITIC      DISCONNECTED
FLOW        FLOW                            VEIN          FLOW

TIME →

*Fig. 4*

OCCLUDED FLOW

0.6

PHLEBITIC VEIN

0.2

FLOW RESISTANCE
(TORR · HR / mL )

INFILTRATION

0.1

NORMAL FLOW

0.01

>0.005

DISCONNECTED FLOW

1 SEC

TIME →

RISE TIME

*Fig. 5*